**Europäisches Patentamt**

**European Patent Office**

**Office européen des brevets**

(19)

(11) Veröffentlichungsnummer: **0 082 937**
**B1**

(12)

# EUROPÄISCHE PATENTSCHRIFT

(45) Veröffentlichungstag der Patentschrift:
**03.10.84**

(51) Int. Cl.³: **C 07 C 11/09**, C 07 C 1/24

(21) Anmeldenummer: **82109948.8**

(22) Anmeldetag: **28.10.82**

(54) **Verfahren zur Herstellung von hochreinem Isobuten durch Dehydratisierung von tertiär-Butanol.**

(30) Priorität: **24.12.81 DE 3151446**

(43) Veröffentlichungstag der Anmeldung:
**06.07.83 Patentblatt 83/27**

(45) Bekanntmachung des Hinweises auf die Patenterteilung:
**03.10.84 Patentblatt 84/40**

(84) Benannte Vertragsstaaten:
**AT BE CH DE FR GB IT LI NL SE**

(56) Entgegenhaltungen:
**DE - A - 2 913 796**
**US - A - 4 155 945**

(73) Patentinhaber: **CHEMISCHE WERKE HÜLS AG, - RSP Patente / PB 15 - Postfach 13 20, D-4370 Marl 1 (DE)**

(72) Erfinder: **Obenaus, Fritz, Dr., Jupiterweg 60, D-4370 Marl (DE)**
Erfinder: **Greving, Bernd, Dr., Leverkusener Strasse 20, D-4370 Marl (DE)**
Erfinder: **Balke, Heinrich, Erlenstrasse 9, D-4352 Herten (DE)**
Erfinder: **Scholz, Bernhard, Dr., Kerkenkamp 4, D-4370 Marl (DE)**

## Beschreibung

Die Erfindung betrifft ein Verfahren zur Herstellung von hochreinem Isobuten durch Dehydratisierung von tert.-Butanol (TBA) in Gegenwart eines stark sauren Kunstharzkationenaustauschers als Katalysator.

Die Dehydratisierung von TBA verläuft reversibel und endotherm. Die Umwandlung zu Isobuten und Wasser nimmt hinsichtlich des chemischen Gleichgewichtes grundsätzlich mit steigender Temperatur zu.

Im technischen Massstab wird daher die Dehydratisierung von TBA überwiegend bei hohen Temperaturen von etwa 180 bis 450°C in Gegenwart von schwach sauren Katalysatoren, wie Kieselgel, Thoriumoxid, Titan(IV)oxid oder insbesondere Aluminiumoxid durchgeführt (DE-OS Nr. 2716132=US-PS Nr. 4036905, DE-AS Nr. 2058478=US-PS Nr. 3665048, US-PS Nr. 2377026). Durch die Anwendung hoher Temperaturen wird zwar ein hoher TBA-Umsatz erzielt, unbefriedigend sind jedoch die beträchtlichen Isobutenverluste durch die Oligomerisierung des Isobutens. Neben der Isobutenoligomerisierung findet darüber hinaus unerwünschterweise eine Isomerisierung des gebildeten Isobutens zu n-Butenen statt (DE-AS Nr. 2058478), die zu weiterem Produktverlust führt und vor allem aber die Abtrennung und Gewinnung von reinem Isobuten besonders nachteilig erschwert.

Wird die Dehydratisierung von TBA bei niedrigeren Temperaturen unter 180°C durchgeführt, so sind aktivere, stark saure Katalysatoren notwendig. Wegen der ihnen anhaftenden Nachteile wie Korrosion, Abwasserprobleme, aufwendige Aufarbeitung usw. verlieren die homogenen sauren Katalysatoren wie z.B. Mineralsäuren, organische Sulfonsäuren und Heteropolysäuren für die Dehydratisierungsreaktion an Bedeutung. Kationenaustauscherharze gewinnen dagegen an Bedeutung als Katalysatoren.

Aufgrund der schlechteren Gleichgewichtsverhältnisse sind bei tieferen Temperaturen nur noch partielle TBA-Umsätze möglich. Höhere Umsetzungsgrade erfordern daher ein kontinuierliches Abtrennen der Reaktionsprodukte.

Die geschieht durch Einstellen niedrigerer Reaktionsdrücke unter 2 bar, so dass das Isobuten aus dem Reaktionsgemisch als niedrigstsiedende Komponente kontinuierlich zusammen mit einem partialdruckmässigen Anteil TBA und Wasser abdestilliert (US-PS Nr. 3256250). Da die ohnehin geringe Bildungsgeschwindigkeit mit steigendem Wassergehalt im Reaktionsgemisch stark abnimmt, eignen sich diese Verfahrensweisen nur für TBA-reiche Reaktionsgemische. Für wasserreichere TBA-Lösungen als Einsatzgemisch sind Verfahrensschritte beschrieben, die die Verwendung von inerten Schleppmitteln für das Reaktionswasser vorschlagen (US-PS Nrn. 3510538 und 4155945). Bei Zusatz von beispielsweise Benzol oder Xylol als Schleppmittel tritt aber gemäss der Lehre der US-PS Nr. 3510538 insbesondere oberhalb von 100°C verstärkt Isobutenoligomerisierung auf. Ausserdem erschwert der Zusatz einer dritten, die Reaktion unterstützende Substanz die Aufarbeitung, da dieser Hilfsstoff wieder ausgetragen und zurückgewonnen werden muss.

Zur Überwindung dieser Nachteile wurde vorgeschlagen, die wässerige TBA-Lösung dem Rektifizierteil einer Kolonne zuzuführen, aus dem heraus bevorzugt bei 85°C und einem Druck von maximal 1,9 bar ein TBA-reicher, wasserhaltiger Dampfstrom durch das oberhalb vom Rektifizierteil in loser Schüttung angeordnete Katalysatorbett getrieben wird (DE-AS Nr. 2454998=US-PS Nr. 4012456). Das Isobuten wird zusammen mit anteiligem TBA und Wasser gasförmig aus dem Reaktionsteil abgezogen. Dieses Verfahren ist zufriedenstellend selektiv, erfordert aber ein speziell geformtes nicht handelsübliches Kationenaustauscherharz.

Ausser der oft komplizierten Reaktionsführung haben all diese Verfahren grundsätzlich einen gravierenden Nachteil:

Isobuten fällt zusammen mit einem partialdruckmässigen Anteil TBA und Wasser gasförmig unter einem Druck an, bei dem es mit in der Technik üblichen Kühlmitteln nicht mehr kondensiert werden kann.

Zur Reingewinnung des Isobutens durch Rektifizierung sind daher aufwendige Komprimierungs- und Kondensationsstufen erforderlich. Um diesen Nachteil zu vermeiden, führt man bei dem Verfahren der DE-OS Nr. 2913796=GB-PS Nr. 2022129 die Dehydratisierung von TBA in Gegenwart von sauren Kationenaustauscherharzen bei einem Druck von mindestens 5 bar durch. Die Temperatur wird entsprechend so hoch gewählt, dass das entstehende Isobuten zusammen mit dem anteiligen TBA und Wasser gasförmig aus der Reaktionszone abgezogen werden kann. Je nach Reaktionsdruck sind hierfür Temperaturen von etwa 180°C oder höher erforderlich, Temperaturen also, die über der Anwendungsgrenze handelsüblicher Kationenaustauscherharze liegen und die daher unter diesen Bedingungen nur eine begrenzte Katalysatorlebensdauer erwarten lassen. Die höheren Temperaturen haben ausserdem eine gegenüber den vorher genannten Verfahren geringere Isobutenselektivität zur Folge. All diesen Verfahren, die den Stand der Technik für die Dehydratisierung von TBA in Gegenwart von sauren Kunstharzaustauschern wiedergeben, ist gemeinsam, dass die Reaktionskomponenten in einer Mischphase, bestehend aus einer gasförmigen und einer flüssigen Phase vorliegen und der Katalysator als dritte, feste Phase vorliegt. Hierdurch ist der Stofftransport an die katalytisch wirksamen Zentren des Katalysators gehemmt, was dazu führt, dass die auf das Reaktorvolumen bezogene Isobutenbildungsrate, d.h. die Raum-Zeit-Ausbeute für Isobuten, unbefriedigend ist.

Für die Durchführung des Prozesses im technischen Massstab werden daher grosse Katalysatormengen benötigt. Dies wirft Probleme auf hinsichtlich der zu verwendenden Vorrichtung und der Wärmezufuhr für die stark endotherme Spaltreaktion, da wegen der geringen Temperaturstabilität der verwendeten Kunstharzaustauscher Wärme nur von einer Wärmequelle vergleichsweise niedriger Temperatur zugeführt werden kann, so dass eine grosse Wärmeübertragungsfläche erforderlich ist.

Zur Verbesserung der Stofftransportbedingungen für die Spaltung wird bei den Verfahren es Standes der Technik mit bewegtem Katalysatorbett gearbeitet. Das Kunstharzgerüst handelsüblicher Kationenaustauscher, in polaren Agenzien wie TBA und Wasser eingesetzt, unterliegt einer starken Aufquellung und Verformung, die die mechanische Stabilität des Katalysators stark beeinträchtigen.

In einem bewegten Katalysatorbett ist daher der Abrieb und das Brechen der Katalysatorpartikel nicht zu verhindern.

Die vorliegende Erfindung soll die geschilderten Nachteile der Verfahren des Standes der Technik vermeiden.

Damit stellt sich die Aufgabe, ein Verfahren zur grosstechnischen Gewinnung von hochreinem Isobuten durch Dehydratisierung von TBA zu finden, das die Dehydratisierung in einem leistungsfähigeren und wirtschaftlicheren Prozess nach einer einfachen Verfahrensweise ermöglicht.

Diese Aufgabe wird erfindungsgemäss entsprechend den Angaben der Patentansprüche gelöst.

Als Einsatzstoff eignet sich sowohl technisches, mit Oligomeren des Isobutens, beispielsweise Diisobuten, verunreinigtes TBA als auch reines TBA. Bevorzugt setzt man TBA/Wasser-Gemische ein wie z.B. die TBA/Wasser-Azeotrope, wie sie bei der destillativen Aufarbeitung anfallen.

Überraschenderweise wird nach dem erfindungsgemässen Verfahren hochreines Isobuten in sehr hoher Raum-Zeit-Ausbeute mit einer Isobutenselektivität von praktisch 100 Mol-% gewonnen, wenn man die Spaltung bzw. Rückspaltung von TBA zu Isobuten und Wasser an einem in einem Festbett angeordneten stark sauren, organischen Ionenaustauscher in homogener und flüssiger Phase durchführt, und der homogene, flüssige Reaktionsaustrag einem vom Reaktionsraum getrennten Destillationsteil zugeführt wird, in dem die Abtrennung des Isobutens und des Wassers vom nicht umgesetzten TBA erfolgt, das als wässerige TBA-Lösung wieder in die Dehydratisierungsstufe zurückgeführt wird. Dies war unerwartet, da in der bereits genannten DE-OS Nr. 2913976, S. 20, Spalten 27 bis 31 beschrieben ist, dass bei zu hohem Druck, d.h. bei Vorliegen einer homogenen Flüssigphase, die Dehydratisierung schwierig zu bewirken ist. Es war daher überraschend, dass nach dem erfindungsgemässen Verfahren in einer homogenen Flüssigphase Isobuten bei diesen Temperaturen in bisher nicht erreichter Raum-Zeit-Ausbeute (RZA) und Selektivität erhalten wird.

Ein weiterer Vorteil des erfindungsgemässen Verfahrens besteht in der Trennung von Reaktionsraum und Isobutenaufarbeitung, wodurch man in der Wahl der optimalen Bedingungen für die Reaktionsstufe und für die Destillationsstufe frei und flexibel ist, was besonders im Hinblick auf die Produktqualität sowie auch im Hinblick auf die Katalysatorstabilität von grossem Vorteil ist.

Die Reaktionstemperatur liegt beim erfindungsgemässen Verfahren bei 80 bis 150°C. Bei erhöhten Temperaturen von z.B. 110°C ist eine adiabatische Fahrweise von Vorteil. Die bevorzugte Temperatur ist 100 bis 130°C, da in diesem Bereich die Isobuten-Raum-Zeit-Ausbeute und die Katalysatorlebensdauer in wirtschaftlicher Hinsicht optimal sind.

Der Reaktionsdruck beträgt 5 bis 25 bar, wobei man den Druck so hoch wählt, dass das im Reaktor entstehende Isobuten ohne Ausbildung einer Gasphase vollständig und homogen im Reaktionsgemisch gelöst bleibt. Im allgemeinen ist es vorteilhaft, wenn der Reaktionsdruck grösser ist als der Kolonnendruck im Aufarbeitungsteil, weil dann zur Unterstützung der Isobutenabtrennung vorteilhafterweise die Entspannungsverdampfung genutzt werden kann.

Der TBA-Gehalt im Einsatzgemisch vor dem Reaktor liegt bei 40 bis 90, bevorzugt bei 55 bis 85 Gew.-%. Das in den Reaktor eingefahrene Einsatzgemisch setzt sich zusammen aus dem frisch zugeführten tert.-Butanol, vorzugsweise als TBA/Wasser-Azeotrop, und dem kreisgeführten TBA/Wasser-Gemisch. Dieses Gemisch fährt man kontinuierlich in den Reaktor.

Die im Einzelfall besonders günstige Raumgeschwindigkeit hängt vom Wassergehalt des Reaktionsgemisches und in hohem Masse von der Reaktionstemperatur und von der Aktivität des verwendeten Katalysators ab und ist für jeden Katalysator individuell zu ermitteln.

Die Raumgeschwindigkeit (LHSV) in Liter Beschickung je Liter aufgequollener Katalysator je Stunde beträgt im allgemeinen bei einer Reaktionstemperatur von 80 bis 100°C 50 bis 100 l/l·, von 100 bis 130°C 100 bis 300 l/l·h und von 130 bis 150°C bis 600 l/l·h.

Doch können auch weniger günstige Raumgeschwindigkeiten gewählt werden. Das Vergleichsbeispiel 1 zeigt die geringere Raum-Zeit-Ausbeute und die deutlich schlechtere Selektivität bei einem zweiphasigen, flüssigen Reaktionsgemisch. Vergleichsbeispiel 2 verdeutlicht die bei einer TBA-Konzentration oberhalb von 90 Gew.-% im Reaktionsgemisch zunehmend schlechtere Isobutenselektivität. Die Vergleichsbeispiele 3 und 4 zeigen insbesondere die geringere Raum-Zeit-Ausbeute und die schlechtere Selektivität, bei einem gasförmig-flüssigen Reaktionsgemisch, aus dem Isobuten gasförmig abgezogen wird.

Als organische Ionenaustauscher sind sulfonierte organische Harze (z.B. nach der US-PS Nr. 2480940 hergestellte) insbesondere die sulfonierten, mit Divinylbenzol vernetzten Polystyrolharze (US-PS Nr. 2922822), die gelartig beschaffen sein können oder zur Vergrösserung der Oberfläche eine Schwammstruktur mit Makroporen (DE-PS Nr. 1224294=GB-PS Nr. 957000) oder zur Erhöhung der Hydrolysestabilität ihrer katalytisch wirksamen Sulfonsäuregruppen ein halogeniertes Kunstharzgerüst (US-PS Nr. 3256250) besitzen können, geeignet. Die handelsüblichen organischen Ionenaustauscher werden bevorzugt in der Wasserstofform verwendet. Aber auch kommerzielle, makroporöse Kationenaustauscher, die durch partiellen Ionenaustausch oder durch thermische Desulfonierung modifiziert sind, können eine zufriedenstellende Aktivität bei einer gleichzeitig höheren Hydrolysestabilität der Sulfonsäuregruppe haben.

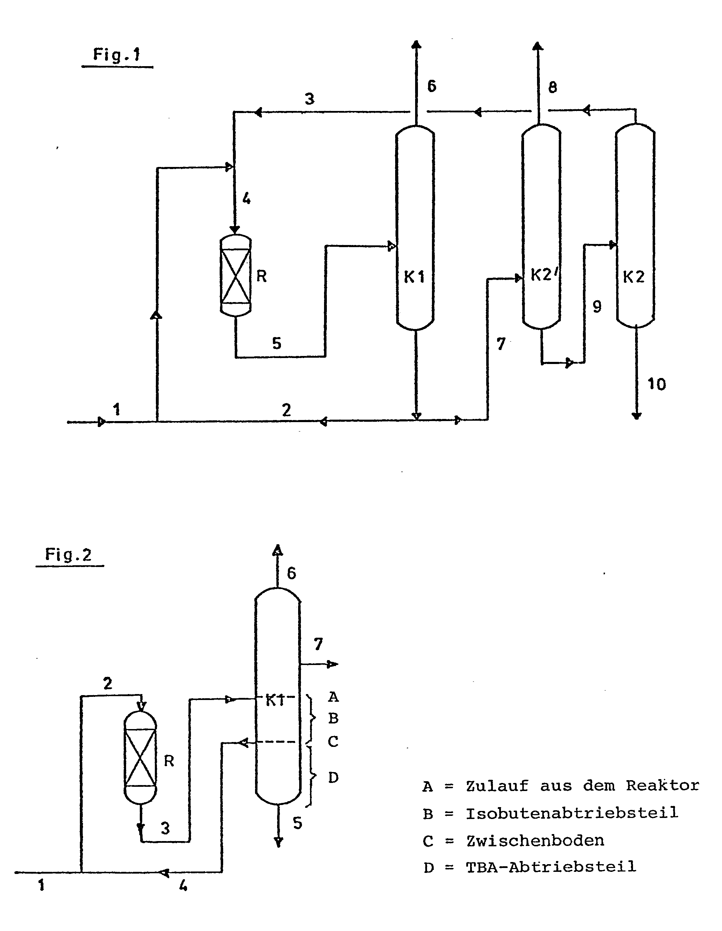

Die Reingewinnung des Isobutens und die Abtrennung des Wassers erfolgt in einem vom Reaktionsraum getrennten Aufarbeitungsteil. Nach einer bevorzugten Ausführungsform des erfindungsgemässen Verfahrens (Abb. 1) wird zur Gewinnung von reinem Isobuten das über Leitung 5 den Reaktor homogen und flüssig verlassende Reaktionsgemisch einer Druckkolonne (K1) zugeführt.

Der Kopfdruck der Kolonne K1 beträgt im allgemeinen 3 bis 10, vorzugsweise 5 bis 7 bar, da in diesem Bereich bei einer Kondensation des Destillats mit Kühlwasser besonders wirtschaftlich destilliert werden kann.

Das Isobuten wird mit den Spuren Wasser, die dem Isobuten/Wasser-Azeotrop entsprechen, über Kopf abdestilliert (Leitung 6). Das Wasser setzt sich in der Vorlage als separate Phase ab und kann problemlos abgetrennt werden. Das als Bodenprodukt anfallende TBA/Wasser-Gemisch enthält im allgemeinen weniger als 0,5 Gew.-% Isobuten und wird in der Hauptmenge über Leitung 2 vor den Reaktor zurückgeführt. Die Sumpftemperatur kann unabhängig von der Reaktoreingangstemperatur so gewählt werden, dass einerseits die Destillation bei wirtschaftlichen Bedingungen durchgeführt werden kann und andererseits die für die Stabilität des Katalysators einzuhaltenden Temperaturen nicht überschritten werden. In dieser Destillationsstufe können selbstverständlich auch niedere und höhere Drücke zur Anwendung kommen, obwohl das aus wirtschaftlichen Gründen weniger zweckmässig ist.

Zur Abtrennung des zum Isobuten in äquivalenter Menge entstehenden Wassers und des gegebenenfalls mit dem frischen TBA eingeschleusten Wassers wird ein Teilstrom von dem Sumpfprodukt über die Leitung 7 der Kolonne K2' zugeführt, in der im Einsatzgemisch enthaltende oder eventuell während der Reaktion in Spuren entstehende Isobutendimere über Kopf (Leitung 8) als ternäres Gemisch mit TBA und Wasser abdestilliert werden. Das Sumpfprodukt wird über Leitung 9 der Kolonne K2 zugeführt, in der als Kopfprodukt ein TBA/Wasser-Gemisch von azeotroper Zusammensetzung abgezogen und über Leitung 3 vor den Reaktor zurückgeführt wird. Im Sumpf der Kolonne K2 wird über Leitung 10 das überschüssige Wasser ausgeschleust. Die Kolonnen K2' und K2 werden bei Normaldruck oder erhöhtem Druck betrieben.

Das ternäre Gemisch vom Kopf der K2' wird zweckmässigerweise mit Wasser zerlegt und die TBA enthaltende wässerige Phase in die Kolonne zurückgefahren. Enthält das Reaktionsgemisch keine Isobutendimere, so entfällt die Kolonne K2'.

Bei Verwendung von TBA/Wasser-Azeotrop (ca. 88 Gew.-% TBA bei Normaldruck) als frisches Einsatzgemisch (Leitung 1) beträgt das Verhältnis der Kreislaufströme aus dem Sumpf der Kolonne K1 und aus dem Kopf der Kolonne K2 vorzugsweise 2 bis 130:1, insbesondere 5 bis 30:1.

Das TBA/Wasser-Gemisch wird vorzugsweise von oben nach unten durch das Katalysatorfestbett des Reaktors R geführt.

Eine weitere erfindungsgemässe Ausführungsform des Verfahrens, die besonders wirtschaftlich ist, ist zur Verdeutlichung in der Abbildung 2 wiedergegeben.

Der homogene, flüssige Reaktionsaustrag wird über Leitung 3 der Druckkolonne K1 zugeführt, in der über Kopf das reine Isobuten als Wasserazeotrop abdestilliert wird. Das mitgeschleppte Wasser scheidet sich als separate Phase in der Vorlage ab. Im Abtriebsteil der Kolonne wird das TBA azeotrop von dem ablaufenden Wasser abdestilliert, so dass am Sumpf der Kolonne K1 das von Isobuten und TBA befreite Wasser über Leitung 5 abgezogen wird. Aus einem Zwischenboden oberhalb des Abtriebsteils, in dem das TBA azeotrop von dem ablaufenden Wasser abdestilliert wird, zweigt man ein mit TBA angereichertes TBA/Wasser-Gemisch als Seitenstromabzug über Leitung 4 ab, mischt es als Kreislaufstrom dem frischen Eduktstrom (Leitung 1) zu und führt es dann über Leitung 2 vorzugsweise von oben nach unten durch den Reaktor. Enthält das Reaktionsgemisch Isobutenoligomere, so können diese durch einen Seitenabzug auf dem Verstärkungsteil der Kolonne K1 über Leitung 7 ausgeschleust werden.

Diese bevorzugte besondere Ausführungsform des Verfahrens ist deshalb besonders wirtschaftlich, weil die Kondensation des TBA/Wasser-Azeotrops bei der Abtrennung des Wassers in einem eigenen Kondensator, wie bei Verwendung der Kolonne K2, entfällt und statt dessen die Kondensationswärme der Auftrennung des energieverbrauchenden Reaktionskreislaufes dient.

Durch das oben beschriebene Verfahren lässt sich in einfacher Weise aus TBA hochreines Isobuten mit einer Selektivität für Isobuten von über 99,9 Mol-% gewinnen. Der Umsetzungsgrad von TBA beträgt praktisch 100%. Da die Reaktion unter milden Bedingungen an einem Katalysatorfestbett durchgeführt wird, erreichen auch handelsübliche, organische Kationenaustauscher technisch zufriedenstellende Katalysatorstandzeiten. Die Umsetzung kann mit kontinuierlichem oder diskontinuierlichem Frisch-TBA-Einsatz in den Reaktionskreislauf erfolgen. Bevorzugt arbeitet man kontinuierlich. Die Reinheit des erhaltenen Isobutens beträgt >99,9%. In der Regel lassen sich Werte bis zu 99,99% erreichen. Der Rest ist Wasser. Das erfindungsgemäss erhaltene hochreine Isobuten verwendet man bevorzugt zur Herstellung von Polyisobuten, Butylkautschuk sowie für Alkylierungen.

Die folgenden Beispiele dienen den Erläuterungen des Verfahrens.

*Beispiel 1:*

Als Katalysator verwendet man ein kommerzielles, stark saures Ionenaustauscherharz (makroporöses, sulfoniertes, mit Divinylbenzol vernetztes Polystyrolharz). Weitere Kenndaten des Harzes sind: BET-Oberfläche ca. 40 m²/g trockenes Harz, Divinylbenzolgehalt ca. 18%, Korngrössenverteilung 0,5 bis 1,3 mm, Wasserstoffionenkapazität ca. 3,8 mEq H$^+$/g trockenes Harz.

Gemäss der Abb. 1 leitet man in den Reaktor R, der mit 78 l des oben beschriebenen Kationenaustauscher-Harzes gefüllt ist, über Leitung (1) 711 kg/h frisches Edukt, in dem 623 kg/h TBA, 85 kg/h Wasser und 2,85 kg/h C$_8$-Olefine enthalten sind, über Leitung (2) 10 455 kg/h aus dem Sumpf der Kolonne K1 zurückgeführtes Kreislaufgemisch, das 6480 kg/h TBA, 3938 kg/h Wasser und 36,9 kg/h C$_8$-Olefine enthält und über Leitung (3) 565 kg/h Kreislaufgemisch aus dem Kopf der Kolonne K2, das 497 kg/h TBA und 68 kg/h Wasser enthält, insgesamt über Leitung (4) 11 731 kg/h Einsatzgemisch, das 64,7 Gew.-% TBA enthält.

Die Spaltreaktion führt man bei 10 bar und einer mittleren Temperatur von 120° C durch.

Das Reaktionsgemisch führt man über Leitung 5 der Kolonne K1 zu und entspannt vor Eintritt auf den Kolonnendruck von 6 bar ($\triangleq$ Kopfdruck). In der Kolonne K1 nimmt man über Kopf (Leitung 6) 470 kg/h reines Isobuten ab. Das Destillat enthält dem Azeotrop entsprechend Wasser, das sich als separate Phase in der Vorlage absetzt und ausgeschleust wird. Das Sumpfprodukt der Kolonne K1 hat die Zusammensetzung 6980 kg/h TBA, 4242 kg/h Wasser und 39,75 kg/h C$_8$-Olefine. Die Hauptmenge wird über Leitung (2) zurückgeführt. Ein Teilstrom, 806 kg/h, wird über Leitung (7) nach Entspannung auf den Kolonnendruck von 1,2 bar ($\triangleq$ Kopfdruck) der Kolonne K2' zugeführt. Über Kopf (Leitung 8) werden in der Kolonne K2' 2,85 kg/h C$_8$-Olefine zusammen mit 2,2 kg/h TBA und 0,65 kg/h Wasser als ternäres Gemisch abgezogen. Das Sumpfprodukt wird über Leitung (9) der Kolonne K2 zugeführt, in der über Kopf (Kopfdruck 1,02 bar) das azeotrope TBA/Wasser-Gemisch abgezogen und über Leitung (3) zurückgeführt wird. Das Verhältnis der Kreislaufströme aus der Kolonne K1 und K2 beträgt 18,5:1. Im Sumpf der Kolonne K2 fallen 235 kg/h Wasser an, die über Leitung (10) ausgeschleust werden. Die Isobutenselektivität beträgt praktisch 100%. Bei Zerlegung des C$_8$/TBA/Wasser-Gemisches vom Kopf der Kolonne K2' mit Wasser und Rückführung der TBA enthaltenden wässerigen Phase beträgt der Umsetzungsgrad von TBA ebenfalls praktisch 100%. Die Isobutenreinheit ist 99,99%.

*Beispiel 2:*

Als Katalysator verwendet man ein kommerzielles, stark saures Ionenaustauscherharz (makroporöses, sulfoniertes, mit Divinylbenzol vernetztes Polystyrolharz) mit folgenden Kenndaten: BET-Oberfläche ca. 25 m²/g trockenes Harz, Divinylbenzolgehalt ca. 8%, Korngrössenverteilung 0,5 bis 1,2 mm, Wasserstoffionenkapazität ca. 4,1 mEq H$^+$/g trockenes Harz.

Gemäss dem Fliessschema in Abb. 2 führt man die TBA-Spaltung bei einer mittleren Temperatur von 120° C und einem Druck von 20 bar wie folgt durch.

In einen Reaktor R, der mit 42 l des oben beschriebenen Katalysators gefüllt ist, führt man mit dem Strom (2) 4161 kg/h TBA, 1040 kg/h Wasser und 3,4 kg/h C$_8$-Olefine ein. Der Strom (2) enthält 80 Gew.-% TBA und setzt sich zusammen aus dem frischen Zustrom (Leitung 1), der 675 kg/h TBA, 92 kg/h Wasser und 2,8 kg/h C$_8$-Olefine enthält, und dem über Leitung 4 aus der Druckkolonne K1 rückgeführten Kreislaufstrom, der 3486 kg/h TBA, 948 kg/h Wasser und 0,6 kg/h C$_8$-Olefine enthält. Der über Leitung 4 rückgeführte Kreislaufstrom enthält 78,6 Gew.-% TBA. Den Reaktoraustrag führt man über Leitung 3 der Kolonne K1 zu und entspannt auf den Kolonnendruck von 7 bar ($\triangleq$ Kopfdruck). In der Kolonne K1 destilliert man über Kopf (Leitung 6) 510 kg/h reines Isobuten ab. Das Destillat enthält dem Azeotrop entsprechend Wasser, das man als separate Phase in der Vorlage abtrennt. Im Sumpf fallen 256 kg/h Wasser an und werden über Leitung 5 ausgeschleust. Aus dem Verstärkungsteil der Kolonne K1 schleust man über einen Seitenabzug 2,9 kg/h C$_8$-Olefine zusammen mit 2,5 kg/h TBA und 0,7 kg/h Wasser aus. Mit dem Wasser aus der Vorlage des Kopfproduktes sowie mit einem Teil des aus dem Sumpf ausgeschleusten Wassers zerlegt man das C$_8$-Olefin/TBA/Wasser-Gemisch und führt die TBA-enthaltende wässerige Phase zurück. Aus dem Abtriebsteil der Kolonne K1 zweigt man über Leitung 4 den Kreislaufstrom als Seitenabzug ab und führt ihn vor den Reaktor zurück. Die Isobutenselektivität beträgt 99,98%, der Umsetzungsgrad für TBA praktisch 100% und die Isobutenreinheit 99,99%.

*Beispiele 3 bis 8:*

In einer Serie weiterer Beispiele dehydratisiert man TBA gemäss der Verfahrensweise nach Beispiel 1 (vgl. Abb. 1). Das als Katalysator verwendete kommerzielle makroporöse, stark saure Ionenaustauscherharz (sulfoniertes, mit Divinylbenzol vernetztes Polystyrolharz) hat folgende Kenndaten: BET-Oberfläche ca. 35 m²/g trockenes Harz, Divinylbenzolgehalt 10%, Korngrössenverteilung 0,5 bis 1,2 mm, Wasserstoffionenkapazität ca. 4,5 mEq H$^+$/g trockenes Harz. Von diesem Harz werden 100 ml in TBA/Wasser-Azeotrop (ca. 12 Gew.-% Wasser) aufgequollen in das untere Viertel eines 100 cm langen Rohrreaktors (Innendurchmesser 2,29 cm) mit Heizmantel eingefüllt. Das Restvolumen ist mit V4A-Füllkörpern aufgefüllt und dient als Vorwärmzone. Im Reaktor ist zentral ein verschiebbares Thermoelement angeordnet. Vor und hinter dem Reaktor sind zur Überprüfung der Homogenität des Flüssigkeitsgemisches Schaugläser angebracht. Das Volumen des Kolonnensumpfes der Kolonnen K1, K2' und K2 wird über eine automatische Niveauregelung konstant gehalten. Über Leitung 1 fährt man frisches, isobutenoligomerfreies TBA/Wasser-Gemisch von azeotroper Zusammensetzung ein. Die angewendeten Bedingungen und die dabei erzielten Ergebnisse sind in der nachstehenden Tabelle 1 zusammengestellt. Die Raumgeschwindigkeit (LHSV) ist in Liter Beschickung je Liter aufgequollener Katalysator je Stunde angegeben, die
Isobuten-Raum-Zeit-Ausbeute (RZA) in kg Isobuten je Liter aufgequollener Katalysator je Stunde. Die Analyse der Mengenströme erfolgte gaschromatographisch. Mit Ausnahme des Beispiels
5 erübrigt sich die Kolonne K2'. Das erhaltene Isobuten hat einen Reinheitsgrad von 99,99%.

*Tabelle 1*

| Beispiel Nr. | Temperatur (°C) | frisches Azeotrop (kg/h) | LHSV (l/l·h) | Verhältnis der Massenströme 1 : 2 : 3 | Strom 4 TBA (Gew.-%) | $H_2O$ (Gew.-%) | Isobuten- RZA (kg/l·h) | Isobuten- Selektivität (Mol-%) |
|---|---|---|---|---|---|---|---|---|
| 3 | 110 | 0,39 | 54 | 1 : 9,3 : 1 | 70 | 30 | 2,6 | 100 |
| 4 | 110 | 0,69 | 140 | 1 : 15,5 : 1,1 | 70 | 30 | 4,6 | 100 |
| 5 | 120 | 0,44 | 45 | 1 : 6,5 : 0,9 | 70 | 30 | 2,9 | 99,9 |
| 6 | 120 | 1,02 | 139 | 1 : 8,2 : 1,3 | 70 | 30 | 6,8 | 100 |
| 7 | 120 | 0,48 | 80 | 1 : 12,6 : 0,6 | 60 | 40 | 3,2 | 100 |
| 8 | 120 | 1,0 | 206 | 1 : 16,0 : 0,6 | 60 | 40 | 6,5 | 100 |

*Beispiel 9:*

Als Katalysator verwendet man das kommerzielle Kationenaustauscherharz aus Beispiel 1. Ein
80 cm langer Rohrreaktor aus Stahl mit 2,29 cm
Innendurchmesser und einem als Thermostat dienenden Mantel, der zwei auf verschiedene Temperaturniveaus beheizbare Zonen hat, ist mit 20 ml in
TBA/Wasser-Azeotrop gequollenem Austauscherharz gefüllt. Das Harz befindet sich im unteren Drittel des Reaktors. Das restliche Reaktorvolumen ist mit V4A-Füllkörpern ausgefüllt und
dient als Vorwärmerzone. Im Reaktor ist zentral ein
verschiebbares Thermoelement angeordnet. Ein
vor und hinter dem Reaktor angebrachtes Schauglas erlaubt die Überprüfung der Homogenität des
Flüssigkeitsgemisches. In einer Serie von Versuchen wird die TBA-Spaltung bei nahezu isothermer Fahrweise (Temperaturdifferenz im Katalysatorbett maximal 2°C) gemäss der Verfahrensweise
nach Beispiel 2, jedoch bei einem Druck von
11 bar, durchgeführt, wobei die Temperatur und
die TBA-Konzentration im Mengenstrom 2 variiert
werden. Das frische TBA wird als isobutenoligomerfreies, reines (99%) TBA über Leitung 1 eingeführt. Die TBA-Konzentration im Seitenstromabzug aus dem Abtriebsteil der Kolonne (Leitung 4)
beträgt bei 60 Gew.-% TBA in Strom 2 ca.
58 Gew.-%, bei 70 Gew.-% TBA ca. 68 Gew.-%,
bei 80 Gew.-% TBA ca. 78 Gew.-%, bei 90 Gew.-%
TBA ca. 85 Gew.-%. Die Mengenströme werden
gaschromatographisch analysiert. Die angewendeten Bedingungen und die dabei erzielten Ergebnisse sind in der Tabelle 2 zusammengestellt. Für
die Raumgeschwindigkeit (LHSV) und Raum-
Zeit-Ausbeute (RZA) gilt die Definition von Beispielen 3 bis 8.

*Beispiel 10:*

Ein makroporöses Ionenaustauscherharz mit
den in Beispiel 1 angegebenen Kenndaten wird
mit 1 mmol/g trockenes Harz Na-Ionen so desaktiviert, dass eine Restwasserstoffionenkapazität von
2,8 mEq H+/g trockenes Harz verbleibt.

In den im Beispiel 9 beschriebenen Reaktor werden 30 ml in TBA/Wasser-Azeotrop aufgequollenes Harz eingefüllt. Die Dehydratisierung des TBA
wird gemäss der in Beispiel 9 angegebenen Verfahrensweise durchgeführt. Die Bedingungen und
die erzielten Ergebnisse sind in Tabelle 3 angegeben.

*Tabelle 2*

| Temperatur (°C) | LHSV (l/l·h) | Isobuten-RZA bei TBA-Gew.-% in Strom 2 (kg/l·h) | | | | Isobuten-Selektivität bei TBA Gew.-% in Strom 2 (Mol-%) | | | |
|---|---|---|---|---|---|---|---|---|---|
| | | 90 | 80 | 70 | 60 | 90 | 80 | 70 | 60 |
| 90 | 120 | — | 2,0 | 1,4 | 1,0 | — | 100 | 100 | 100 |
| 100 | 120 | — | 4,1 | 3,0 | 2,2 | — | 100 | 100 | 100 |
| 116 | 120 | — | 7,3 | 6,0 | 4,8 | — | 100 | 100 | 100 |
| 120 | 120 | — | 15,1 | 12,0 | 9,8 | — | 99,9 | 100 | 100 |
| 130 | 300 | — | 22,6 | 19,2 | 15,3 | — | 99,9 | 99,9 | 100 |
| 140 | 300 | 40,5 | — | — | — | 99,8 | — | — | — |

Die Isobutenreinheit beträgt 99,99%.

*Tabelle 3*

| Temperatur (°C) | LHSV (l/l·h) | Isobuten RZA bei TBA Gew.-% in Strom 2 (kg/l·h) | | |
|---|---|---|---|---|
| | | 90 | 70 | 60 |
| 100 | 100 | — | 2,5 | 1,9 |
| 110 | 120 | — | 5,2 | 4,1 |
| .120 | 130 | — | 10,5 | 9,2 |
| 140 | 300 | 28,3 | — | — |

Die Isobutenselektivität beträgt in allen Fällen 100 Mol-% und die Isobutenreinheit 99,99%.

*Vergleichsbeispiel 1*

Dieses Beispiel zeigt die Aktivität und Selektivität des Katalysatorharzes bei einem zweiphasigen, flüssigen Reaktionsgemisch. Katalysator und Versuchsdurchführung entsprechen den Angaben in den Beispielen 3 bis 8. Bei einer mittleren Reaktionstemperatur von 130°C, einem Reaktionsdruck von 15 bar und einer Raumgeschwindigkeit von 120 l/l·h werden 0,16 kg/h frisches Azeotrop über Leitung 1 zusammen mit den Kreislaufströmen aus Leitung 2 und 3 von oben nach unten durch den Reaktor gefahren. Das Verhältnis der Massenströme aus Leitung 1, 2 und 3 ist 1:64,5:0,12. Das TBA/Wasser-Gemisch am Reaktoreingang enthält 25 Gew.-% TBA und 755 Gew.-% Wasser. Der Reaktoraustrag ist zweiphasig und flüssig. Die Isobuten-Raum-Zeit-Ausbeute beträgt 1,1 kg/h, die Isobutenselektivität 97,8 Mol-%.

*Vergleichsbeispiel 2:*

Gemäss der in Beispiel 9 beschriebenen Versuchsdurchführung wird über Leitung 2 (vgl. Abb.

2) ein TBA/Wasser-Gemisch mit 95 Gew.-% TBA und 5·Gew.-% Wasser bei 150°C, einer Raumgeschwindigkeit von 120 l/l·h und einem Reaktionsdruck von 15 bar durch den Reaktor gefahren. Der Reaktionsaustrag ist homogen und flüssig. Die Isobuten-Raum-Zeit-Ausbeute beträgt 21,6 kg/h, die Isobutenselektivität 98,6 Mol-%.

*Vergleichsbeispiele 3 und 4:*

Die Versuchsdurchführung erfolgt mit folgenden Abänderungen nach Beispiel 2. 100 ml Kationenaustauscherharz aus Beispiel 1, in TBA/Wasser-Azeotrop gequollen, befinden sich in loser Schüttung in einem 80 cm langen Glaseraktor (Innendurchmesser 4,5 cm) mit einem verschiebbaren Thermoelement in der Mitte und einem Heinzmantel. Der Reaktionsdruck wird so gewählt, dass bei der betreffenden Reaktionstemperatur das Reaktionsgemisch siedet. Das frische, isobutenoligomerfreie TBA/Wasser-Gemisch von azeotroper Zusammensetzung wird den kreisgeführten TBA/Wasser-Gemischen zugemischt und von unten nach oben durch den Reaktor geführt. Die hierdurch und durch das Verdampfen des gebildeten Isobutens hervorgerufene Turbulenz hält den Katalysator in Dispersion. Das gebildete Isobuten wird über eine am Kopf des Reaktors befindliche Glockenbodenkolonne (10 Böden) kontinuierlich gasförmig abgezogen. Die flüssige Reaktionsphase wird zum Abtrenner des Katalysators am oberen Reaktorende über eine Filterkerze aus dem Reaktionsraum (Volumen 1,2 l) abgezogen und analog der in Beispiel 2 beschriebenen Verfahrensweise behandelt. Die angewendeten Bedingungen und die erzielten Ergebnisse zeigt Tabelle 4. Die Raumgeschwindigkeit und die Raum-Zeit-Ausbeute ist jeweils auf das gequollene Katalysatorvolumen bezogen. Die Mengenströme wurden gaschromatographisch analysiert.

*Tabelle 4*

| Vergleichs-beispiel Nr. | Temperatur (°C) | Reaktions-druck (bar) | LHSV (l/l·h) | Gew.-% im Strom Isobuten vor dem Reaktor | | RZA (kg/l·h) | Selektivität (Mol-%) |
|---|---|---|---|---|---|---|---|
| | | | | TBA | $H_2O$ | | |
| 1 | 80 | 1,05 | 90 | 80 | 20 | 0,32 | 99,7 |
| 2 | 90 | 1,4 | 110 | 80 | 20 | 0,75 | 99,5 |

## Patentansprüche

1. Verfahren zur Herstellung von hochreinem Isobuten durch Dehydratisierung von tert.-Butanol in Gegenwart eines Säurekatalysators, dadurch gekennzeichnet, dass man

a) tertiär Butanol in einer wässerigen Lösung, die 40 bis 90 Gew.-% tert.-Butanol enthält, kontinuierlich in einen Reaktor einführt und in homogener und flüssiger Phase an einem Katalysatorfestbett, bestehend aus stark saurem Ionenaustauscherharz, bei einer Temperatur von 80 bis 150°C und einem Druck von 5 bis 25 bar dehydratisiert,

b) anschliessend das homogene, flüssige Reaktionsgemisch in einem vom Reaktionsraum getrennten Destillationsteil fraktioniert und das Isobuten vom Wasser und dem nicht umgesetzten tert.-Butanol abtrennt,

c) dann das nicht umgesetzte tert.-Butanol enthaltende tert.-Butanol/Wasser-Gemisch nach destillativer Anreicherung des tert.-Butanols in einem so gewählten Kreislaufmengenstrom wieder dem Reaktor zuführt, dass das Gemisch aus dem frisch zugeführten tert.-Butanol und dem kreisgeführten tert.-Butanol/Wasser-Gemisch am Reaktoreingang 40 bis 90 Gew.-% tert.-Butanol enthält und das Wasser, das zum gebildeten Isobuten in äquivalenter Menge entsteht und gegebenenfalls dem Reaktionskreislauf mit frischem tert.-Butanol zugeführt wird, ausschleust.

2. Verfahren nach Anspruch 1, dadurch gekennzeichnet, dass man tert.-Butanol bei einer Temperatur von 100 bis 130°C dehydratisiert.

3. Verfahren nach den Ansprüchen 1 und 2, dadurch gekennzeichnet, dass das gesamte Gemisch aus tert.-Butanol und Wasser am Reaktoreingang 55 bis 85 Gew.-% tert.-Butanol enthält.

4. Verfahren nach den Ansprüchen 1 bis 3, dadurch gekennzeichnet, dass man in einer Destillationskolonne das Isobuten über Kopf vom tert.-Butanol/Wasser-Gemisch abtrennt, im Abtriebsteil der Kolonne das tert.-Butanol azeotrop von dem ablaufenden Wasser abdestilliert, wobei im Sumpf der Kolonne das von Isobuten und tert.-Butanol befreite Wasser anfällt, das ausgeschleust wird, und aus einem Zwischenboden oberhalb des Abtriebsteils, in dem das tert.-Butanol azeotrop von dem ablaufenden Wasser abdestilliert wird, ein mit tert.-Butanol angereichertes tert.-Butanol/Wasser-Gemisch als Seitenstromabzug abzweigt, den man als Kreislaufstrom direkt wieder dem Reaktor zuführt.

## Revendications

1. Procédé de préparation d'isobutène très pur, par déshydratation de butanol tertiaire en présence d'un catalyseur acide, caractérisé par le fait que

a) dans un réacteur, on introduit de façon continue du butanol tertiaire en solution aqueuse, qui contient de 40 à 90% en poids de butanol tertiaire, et on déshydrate en phase homogène et liquide sur une couche fixe de catalyseur formé de résine échangeuse d'ions fortement acide, à une température de 80 à 150°C et sous une pression de 5 à 25 bar,

b) ensuite, on fractionne le mélange réactionnel liquide, homogène, dans une partie de distillation séparée de la chambre de réaction et on sépare l'isobutène de l'eau et du butanol tertiaire non converti,

c) après avoir enrichi le butanol tertiaire par distillation, on délivre à nouveau au réacteur le mélange de butanol tertiaire et d'eau contenant du butanol tertiaire non converti, avec un débit de recyclage choisi de telle façon que le mélange formé du butanol tertiaire nouvellement amené et du mélange butanol tertiaire/eau contienne, à l'entrée du réacteur, de 40 à 90% en poids de butanol tertiaire et on élimine l'eau qui est formée en quantité équivalente à l'isobutène formé et qui est éventuellement amenée au recyclage de réaction avec du butanol tertiaire frais.

2. Procédé selon la revendication 1, caractérisé par le fait que l'on déshydrate le butanol tertiaire à une température de 100 à 130°C.

3. Procédé selon les revendications 1 et 2, caractérisé par le fait que la totalité du mélange de butanol tertiaire et d'eau contient, à l'entrée du réacteur, de 55 à 85% en poids de butanol tertiaire.

4. Procédé selon les revendications 1 à 3, caractérisé par le fait que, dans une colonne à distiller, on sépare à la tête l'isobutène du mélange butanol tertiaire/eau, que dans la section d'épuisement de la colonne on sépare par distillation azéotropique le butanol tertiaire de l'eau qui s'écoule, l'eau débarrassée d'isobutène et de butanol tertiaire se retrouvant dans le bas de la colonne et étant éliminée, et que d'un plateau intermédiaire, au-dessus de la section d'épuisement, on détourne un mélange butanol tertiaire/eau enrichi en butanol tertiaire que l'on ramène directement au réacteur en tant que courant de recyclage.

## Claims

1. A process for the production of high-purity isobutene by dehydration of tert.-butanol in the presence of an acid catalyst, characterised in that

(*a*) tert.-butanol is charged continuously to a reactor in an aqueous solution containing 40 to 90 wt.% tert.-butanol and is dehydrated in a homogeneous liquid phase at a temperature of 80 to 150°C and a pressure of 5 to 25 bar in a solid catalyst bed of strongly acidic ion-exchange resin,

(*b*) subsequently the homogeneous liquid phase is fractionated in a distillation region separated from the reaction chamber and isobutene is separated from water and unconverted tert.-butanol,

(*c*) then a tert.-butanol/water mixture containing unconverted tert.-butanol is recycled to the reactor after being enriched in tert.-butanol by distillation, the amount of the recycle stream being selected so that the mixture of freshly charged tert.-butanol and recycled tert.-butanol/water mixture at the inlet to the reactor contains 40 to 90 wt.% tert.-butanol, and the water which is formed in an amount equivalent to the isobutene and which is optionally supplied to the recycle stream with fresh tert.-butanol is discarded.

2. A process according to Claim 1, characterised in that tert.-butanol is dehydrated at a temperature of 100 to 130°C.

3. A process according to Claims 1 and 2, characterised in that the entire mixture of water and tert.-butanol at the reactor inlet contains 55 to 85 wt.% tert.-butanol.

4. A process according to any of Claims 1 to 3, characterised in that the isobutene is separated from the tert.-butanol/water mixture at the head of a distillation column, the tert.-butanol is azeotropically distilled from the water in the distillation zone of the column so that the water freed from the isobutene and tert.-butanol falls to the sump of the column and is discarded there, and a tert.-butanol/water mixture enriched in tert.-butanol is withdrawn as a sidestream from an intermediate plate above the distillation zone for recycling direct to the reactor as the recycle stream.

0 082 937

**Fig.1**

**Fig.2**

A = Zulauf aus dem Reaktor

B = Isobutenabtriebsteil

C = Zwischenboden

D = TBA-Abtriebsteil